# EUROPEAN PATENT SPECIFICATION

(11) **EP 4 049 697 B1**
(45) Date of publication and mention of the grant of the patent: **17.12.2025**
(21) Application number: 22155268.0
(22) Date of filing: 04.02.2022
(51) Int. Cl.: A61M 5/142, A61M 39/08, F04B 43/00, F04B 43/08, A61J 1/06, A61J 1/10, A61M 5/36, A61M 5/168

(54) **PERISTALTIC INFUSION PUMP TUBE SEGMENT AND INFUSION PUMP DEVICE WITH SUCH A TUBE SEGMENT**
SCHLAUCHSEGMENT EINER PERISTALTISCHEN INFUSIONSPUMPE UND INFUSIONSPUMPENVORRICHTUNG MIT EINEM SOLCHEN SCHLAUCHSEGMENT
SEGMENT DE TUBE DE POMPE À PERFUSION PÉRISTALTIQUE ET DISPOSITIF DE POMPE À PERFUSION DOTÉ D'UN TEL SEGMENT DE TUBE

(30) Priority: 04.02.2021 EP 21020053
(43) Date of publication of application: 31.08.2022
(73) Proprietor: Micrel Medical Devices S.A., Konstantinoupoleos 42 Karela Industrial Area 19441 Koropi (GR)
(72) Inventor: Tsoukalis, Achilleas, Papagou 15669 Attiki (GR)
(74) Representative: Eisenführ Speiser

(56) References cited:
- EP-A1- 2 075 468
- EP-A1- 2 659 923
- EP-A2- 0 858 812
- WO-A1-94/28309
- WO-A2-2011/008624
- US-A- 5 088 522
- US-A1- 2009 053 084
- US-B1- 6 203 528

## Description

### BACKGROUND OF THE INVENTION

Ambulatory infusion pumps are used outside and inside the hospital, for smaller volume drug delivery like 100 ml morphine to palliative care patients, or higher volume drug delivery like in case of parenteral nutrition where large volumetric pumps (LVP) are too big to carry. A big use of ambulatory infusion pumps is with chronic patients at home or with therapies that last long with the patient at home.

False alarms of infusion pumps are very problematic but frequent. It is the aim of the present invention to provide means to reduce false alarms.

US 6,203,528 B1 defining the closest prior art from which the present invention proceeds discloses a unitary molded elastomer conduit for use with a medical infusion pump. The molded conduit includes a plurality of elongated tubular sections defining a continuous lumen therethrough. A first interior surface of the molded conduit includes an interior region having surface characteristics for clamping off the lumen to stop the flow of fluid transport, or for clamping the lumen for use with the peristaltic mechanism of the medical infusion pump. Another interior surface is provided in the molded conduit which is associated with another of the plurality of elongated sections of the integral elastomer conduit. The second interior surface defines a second interior region having surface characteristics for coupling ultrasound across the conduit traversed to the lumen in order to detect gas entrainment and the like. The second interior region thus provides a textured surface relative to the first interior region to direct the signal energy from the signal coupled across the conduit traversed to the lumen.

Accuracy of infusion pumps is far lower than required, since mostly PVC tubes are used which show the required accuracy for 30 minutes after start of infusion but deteriorate largely after several hours or days of infusion. It is the aim of the present invention to increase and keep the required accuracy of the pump very long after start of infusion.

### BRIEF DESCRIPTION OF THE INVENTION

In order to achieve the aforementioned and further objects, according to a first aspect of the present invention defined in claim 1, there is provided a peristaltic infusion pump tube segment having an upstream inlet and a downstream outlet and adapted to be temporally squeezed at at least one squeezed point to be generated by an engagement structure and to be moved towards said outlet for passing a medical fluid from said inlet to said outlet, comprising a first tube portion next to said inlet and having a surrounding wall which is adapted for an upstream occlusion detection, a second tube portion downstream of said first tube portion and adapted to be temporally engaged by the engagement structure, wherein said second tube portion comprises a surrounding wall being at least partly thicker than the wall of said first tube portion, a third tube portion downstream of said second tube portion and having a surrounding wall which is adapted for an air in line detection, and a fourth tube portion next to said outlet and adapted for a downstream pressure detection, characterized in that the wall of said first tube portion has a thickness which is dimensioned so as to allow a detection unit to detect even the smallest possible upstream occlusion, the wall of said second tube portion is thicker than the wall of said first tube portion and is essentially unpolished, the wall of said third tube portion either comprises at least one flat wall portion which is configured for the air in line detection in particular by means of at least one ultrasound sensor, or has a rectangular or square section at least at its external side and comprises flat wall portions configured for the air in line detection in particular by means of at least one ultrasound sensor, wherein the outer surface of the at least one flat wall portion is polished, said fourth tube portion comprises an essentially flat compartment configured for the downstream pressure detection, wherein said flat compartment comprises a disc-like element having essentially an at least part-circle or part-elliptical shape with a diameter or width being larger than the diameter or width of said first tube portion and/or said second tube portion and/or said third tube portion.

According to the first aspect, the wall of said first tube portion has a thickness which is dimensioned so as to allow a detection unit to detect even the smallest possible upstream occlusion.

Further, according to the first aspect, the wall of said second tube portion is thicker than the wall of said first tube portion and is essentially unpolished.

Moreover, according to the first aspect, the wall of said third tube portion either comprises at least one flat wall portion which is configured for the air in line detection in particular by means of at least one ultrasound sensor or has a rectangular or square section at least at its external side and comprises flat wall portions configured for the air in line detection in particular by means of at least one ultrasound sensor, wherein
the outer surface of the at least one flat wall portion is at least partly polished.

Finally, according to the first aspect, said fourth tube portion comprises an essentially flat compartment configured for the downstream pressure detection, wherein
said flat compartment comprises a disc-like element having essentially an at least part-circle or part-elliptical shape with a diameter or width being larger than the diameter or width of said first tube portion and/or said second tube portion and/or said third tube portion.

Preferred embodiments and modifications of the first aspect of the present invention are defined in the dependent claims 2 to 4.

Preferably, the peristaltic infusion pump tube segment is, at least partly, made of silicone, in particular by a silicone molding process, preferably by a silicone injection molding process.

According to a further preferred embodiment of the first aspect, the wall of said second tube portion is made of an elastic material which allows said second tube portion to be essentially reformed at those portions which are currently not subject to the engagement by the engagement structure.

According to a further preferred embodiment of the first aspect, the wall of said second tube portion is configured to allow a viscous fluid to be passed through by suction effect.

According to a further preferred modification of the above embodiment, said flat compartment comprises a long side extending in the direction of the peristaltic infusion pump tube segment.

In order to achieve the aforementioned and further objects, according to a second aspect of the present invention defined in claim 5, there is provided an infusion pump device comprising a pump mechanism module configured as a peristaltic mechanism and including the peristaltic infusion pump tube segment according to the first aspect, wherein the upstream inlet of said peristaltic infusion pump tube segment is adapted to be fluidly connected to an outlet of a medication reservoir.

Preferred embodiments and modifications of the second aspect are defined in the dependent claims 6 to 16.

According to a preferred embodiment of the second aspect, said pump mechanism module is configured as a linear peristaltic mechanism with said peristaltic infusion pump tube segment having an essentially elongated shape.

According to a further preferred embodiment of the second aspect, said peristaltic infusion pump tube segment is replaceable and/or disposable.

According to a further preferred embodiment of the second aspect, the infusion pump device comprises an upstream pressure sensor provided at said first tube portion.

According to a further preferred embodiment of the second aspect, said pump mechanism module comprises an engagement structure adapted to generate at least one squeezed point in said second tube portion and to move it in the direction towards said outlet of said peristaltic infusion pump tube segment.

According to a further preferred embodiment of the second aspect, said engagement structure comprises a plurality of engagement units arranged side by side along the length of said second tube portion of said peristaltic infusion pump tube segment, wherein each engagement unit comprises a follower head which is movable at an angle, in particular a right angle, relative to said second tube portion and adapted to be brought into engagement with said second tube portion in order to squeeze it, wherein said engagement structure is further configured so that the follower heads are temporally brought into engagement with said second tube portion one after another.

According to a modification of the above embodiment, at least one engagement unit comprises a support, a follower body moveably mounted at said support and provided with the follower head, a ball bearing rotatably mounted at said follower body, a cam rotatably mounted at said support and in sliding engagement with said ball bearing, and a spring biasing said moveable follower body with said follower head away from said second tube portion of said peristaltic infusion pump tube segment.

According to a further modification of the above embodiment, said engagement structure comprises a drive unit, wherein the cam of each equipment unit is fixedly mounted to a common rotary shaft which is rotated by said drive unit, and wherein the rotational angle offset of the cam relative to said rotary shaft increases from engagement unit to engagement unit so that the follower heads are temporally brought into engagement with said second tube portion of said peristaltic infusion pump tube segment from engagement unit to engagement unit.

According to a still further modification of the above embodiment, the follower head is mounted upon a stem fixed to the follower body and the engagement unit further comprises a sealing membrane with an opening through which said stem extends in an at least essentially sealing manner.

According to a further preferred embodiment of the second aspect, the infusion pump device comprises a first air in line sensor, in particular an air in line ultrasound sensor, provided at said third tube portion.

According to a further preferred embodiment of the second aspect, the infusion pump device comprises a downstream pressure sensor provided at said fourth tube portion.

According to a further preferred embodiment of the second aspect, the infusion pump device comprises an air eliminating filter which is in fluid communication with the outlet of said peristaltic infusion pump tube segment and a second air in line sensor adapted to detect air in a fluid path downstream of said air eliminating filter.

So, according to a preferred embodiment of the present invention, the pump uses a linear peristaltic structure reducing friction with a ball bearing at each follower. The total construction of the infusion mechanism results in a small size weight and friction while reducing noise to minimum. The followers preferably comprise springloaded side walls, so that there is no kink at the sides during infusion, and are also subject to a removing action without friction by use of the same ball bearing resulting in squeezing the infusion tube through the same follower, wherein each follower comprises a runner at each side so as to slip without friction with side followers. In particular, the whole mechanism and motor assembly is included in one independent part comprising only one rod for assembly and a screw on the other side to adjust the distance from a pressure plate so as to operate with nominal pressure. In particular, the screw is adapted to adjust a squeezing gap in the infusion pump tube segment resulting in an adjustment of the maximum downstream occlusion pressure the infusion pump tube segment can withstand before leaking backwards, at nominal value. So, any contact points with the pump body are minimal, and also the motor and mechanism noise is not transmitted thought the body to the air, a very important feature for infusion pumps. According to a preferred embodiment, the mechanism has a silicone liquid ingress sealing membrane provided not over the followers as in prior art but at an intermediate point below their edge.

According to a preferred embodiment, the infusion segment is made by a relatively new silicone injection molding so that the accuracy tolerance is below 3% and is kept during all infusion time since silicone does not essentially change its form or shape after being squeezed by the followers. Injection molded silicone tubes have also a better accuracy than standard extruded silicone tubes showing large dimensional variations during extrusion.

According to a preferred embodiment, since an injection silicone is used that is formed by a mold, the infusion pump tube segment is provided with several tube portions which are better adapted to each function as follows:
- Upstream there is a reduced thickness tube portion so that an upstream pressure sensor can sense even the smallest upstream occlusion (resulting in a reduction of this tube portion) since it is important to sense it as early as possible.
- Subsequently there is a second normal infusion portion having thicker walls to increase the accuracy with higher suction so that it is reformed when the follower is removed, and to eliminate the need of a squeezing spring plate normally used in infusion pumps with a PVC infusion segment. This tube portion is not polished to reduce friction and power consumption.
- Subsequently there is a third tube portion comprising a polished square section with flat outer walls for a better and secure air in line ultrasound reading, wherein ultrasound plates are best coupled to the tube.
- Subsequently there is fourth tube portion including a flat bottom compartment having the shape of a larger diameter disc that when pressurized exercises a higher force to a downstream pressure sensor against it (F=P*S), a feature that is needed to sense accurately the downstream pressure from where important observations like the possibility of occlusion in a central venous catheter used by chronic patients are deduced.

Preferably, the wall of said first tube portion can have a thickness of about 0.5 mm, whereas the wall of said second tube portion can have a thickness of about 1.0 mm.

According to a further preferred embodiment, said first tube portion has an inner diameter of about 2.5 mm and an outer diameter of about 4 mm, said second tube portion has an inner diameter of about 2 mm and an outer diameter of about 4 mm and said third portion has an inner diameter of about 2 mm and an outer width of about 4 mm.

Preferably, the infusion pump tube segment comprises at least partly a shore hardness of about 50.

The pump preferably comprises means to reduce false alarms for air in line which are very often in hospital and home settings and are very annoying. A further preferred embodiment of the present invention uses a first air in line sensor downstream the pumping mechanism on the infusion pump tube segment so to sense the absence of liquid which leads to a bag empty alarm and a second air in line sensor downstream of an air eliminating filter put inside the drug compartment, so to eliminate the alarm generated by the first air in line sensor if no air is detected downstream and only bubbles but not full air is present at the first air in line detector. Also, preferably, it is detected if a filter is defective, and alarms for the defective filter are generated, if bubbles are detected by the second detector.

### BRIEF DESCRIPTION OF THE DRAWINGS

- Fig. 1: shows a perspective top view (a) and a perspective side view (b) of a pump according to a preferred embodiment.
- Fig. 2: shows the pump of Fig. 1 with its drug bag compartment being opened.
- Fig. 3: shows a portion of the pump of Fig. 1 with its battery and GSM SIM card compartment.
- Fig. 4: shows a peristaltic infusion pump tube segment to be provided within the pump of Fig. 1 in a perspective view (a) and a cross-sectional perspective view (b).
- Fig. 5: shows a perspective view of a pump mechanism module as part of the pump of Fig. 1.
- Fig. 6: shows as a part of the pump mechanism module module of Fig. 5 a follower unit comprising a support with a cam and a follower movably provided at the support and including a ball bearing (a) and said follower individually shown from the opposite side (b).
- Fig. 7: shows an embodiment of a drug bag with an RFID tag prefilled by a pharmaceutical company and including e.g. analgesics.

### DESCRIPTION OF A PREFERRED EMBODIMENT

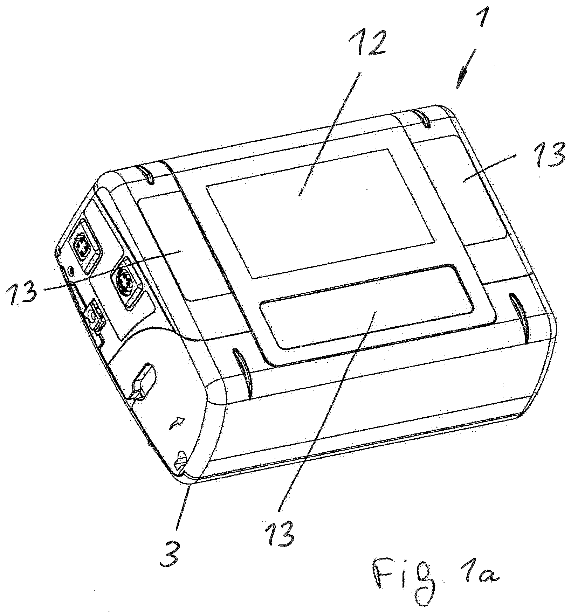

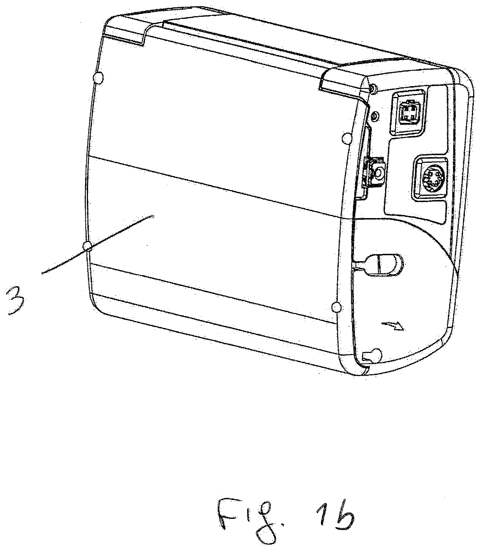

The pump 1 according to a preferred embodiment as shown in the Figs. 1 to 3 includes a removable and exchangeable clear plastic drug bag compartment 4 with a lid 3 lockable by a key mechanism 7, and is embodied in several sizes depending on the therapy used so as to keep the size minimal for each therapy.

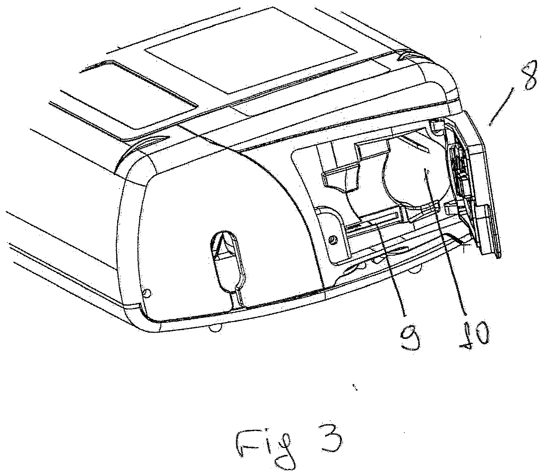

As to be seen from Fig. 3, the pump 1 comprises a battery compartment 10 with a disposable or rechargeable main battery and an internal rechargeable secondary battery (not shown). Two alkaline batteries in series are used as standard, but alternatively e.g. a rechargeable LiPo battery pack with separate connecting pins can be used (not shown), so that it is not possible to recharge alkaline batteries and, hence, to avoid errors. A battery door 8 when open allows access to a SIM card 9 for a GSM communication module (not shown).

The pump 1 additionally includes a smaller safety rechargeable battery (not shown), so that the pump 1 can even run during a battery change and alarm when the main battery has been depleted. This secondary battery is recharged by the main battery or an external power pack cable which is to be connected to the pump 1 and also charges the battery pack, while an external charger for replacement battery packs also exists.

The pump 1 includes a dual microcontroller electronic control for redundancy safety (not shown), and, as schematically depicted in Fig. 1a, further comprises an LCD display 12 and keys 13 with backlight, wherein the dual microcontroller electronic control is further adapted to control said display 12 and said keys 13 and optionally further components not shown.

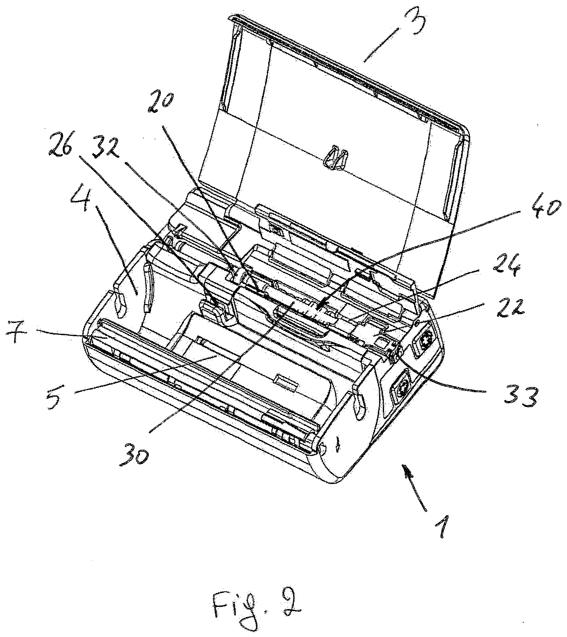

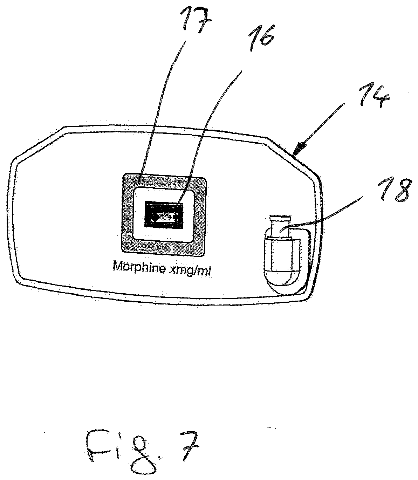

As also shown in Fig. 2, the pump 1 further comprises an RFID reader 5 at the bottom of the bag compartment 4, so that any tagged bag 14, as exemplarily shown in Fig. 7, which bag is provided with a tag or label 16 and an outlet 18 and is put into the compartment 4, can be read so as to make an infusion protocol selection automated and exclude medication errors. According to a system disclosed in EP 2 659 923 A1, an analgesia bag prefilled by a pharmaceutical company for this pump 1 is read automatically with the drug name and concentration guiding the pump 1 to find the correct protocol in an embedded or cloud-based drug library, by passing several steps of programming making it easy for nurses at home who are not trained on this pump 1 to perform an infusion excluding errors in preparation and programming, wherein there may be also the need of a second nurse (mandatory for safety in some countries).

According to the preferred embodiment shown in Fig. 7, the tag 16 on the pharmaceutical bag 14 is put (not with adhesive but) inside a pocket 17 formed by the same bag material, which pocket is thermally bonded on the bag material enclosing the tag with its antenna. The bag 14 is printed with written contents externally as usual on the flip side as also to be seen from Fig. 7. This solution is advantageous to avoid contamination of the drug with adhesive glue material. The outlet 18 of the drug bag 14 preferably includes a valve (not shown) which is opened by an infusion set connector (not shown), so that the drug bag 14 sold through pharmacies needs an infusion set sold through a pump 1 distribution network so as to connect with its outlet 18, also having infusion segment and an downstream accessories like filters and extension line tubing. This is a drug-device combination for a pharmaceutical product dedicated to a specific pump.

In order to help verify infusion and alarm conditions, the pump 1 includes an upstream pressure sensor 20 and a downstream pressure sensor 22, as to be seen from Fig. 2. As further shown in Fig. 2, an infusion pump segment 30 is inserted into the pump 1. The infusion pump segment 30 is disposable and forms a fluidic path from the outlet 18 of the drug bag 14 (Fig. 7) when inserted into the drug bag compartment 4 to an outlet. The infusion pump tube segment 30 comprises an upstream inlet 32 which is adapted to be coupled to the outlet 18 of the drug bag 14 by means of a fluid connector or connecting tubing (not shown) and further comprises a downstream outlet 33 for outputting a fluid drug or medication from the drug bag 14. As further to be seen from Fig. 2, the infusion pump tube segment 30 is to be arranged so that the upstream pressure sensor 20 is provided near the inlet 32 of the infusion pump tube segment 30 and the downstream pressure sensor 22 is provided near the outlet 33 of the infusion pump tube segment 30.

As further shown in Fig. 2, the pump 1 comprises a pump mechanism module 40 which is configured as a linear peristaltic mechanism and adapted to engage a portion of the infusion pump tube segment 30 so as to generate at least one squeezed point in said tube portion and to move it in the direction towards the outlet 33 of the infusion pump tube segment 30. So, the infusion pump tube segment 30 is to be arranged within the pump 1 so as to be subject by engagement of the pump mechanism module 40.

The pump 1 comprises means to reduce false alarms for air in line; these are very often in hospitals and home settings and are very annoying. In the embodiment shown in Fig. 2, said means comprise an air in line detector or sensor 24 downstream of the pump mechanism module 40 at the infusion pump tube segment 30 so as to sense the absence of liquid causing a bag empty alarm, and an additional air in line detector or sensor 26 after an air eliminating filter (not shown) arranged inside the drug compartment 4, so as to eliminate the alarm generated by said air in line sensor 24 if no air is detected downstream and only bubbles but not full air is present at said air in line sensor 24. The additional air in line sensor 26 is provided for detecting air after the air eliminating filter in a connecting tubing (not shown) to be positioned in the drug compartment 4 and connecting the outlet 18 of the drug bag 14 to the inlet 32 of the infusion pump tube segment 30 in some models for therapies like parenteral nutrition which usually generate a lot of air bubbles caused by an emulsion. There is also a detection if a filter is defective (not shown), and in said case alarms are given for a defective filter if bubbles are detected by the additional air in line sensor 26 preferably provided as mentioned above.

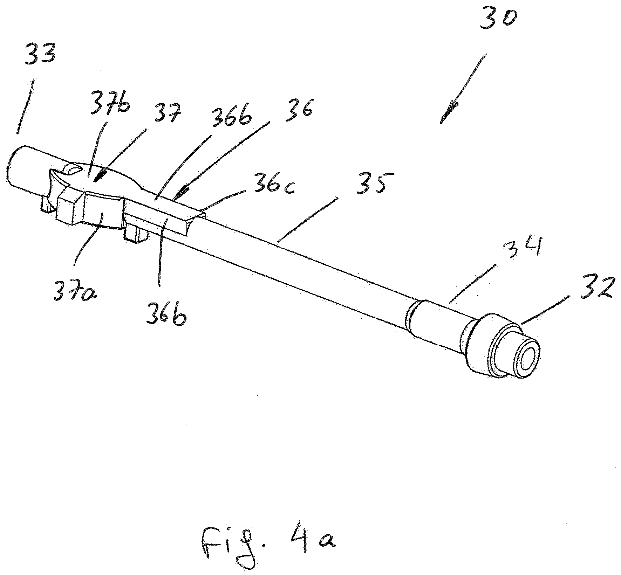

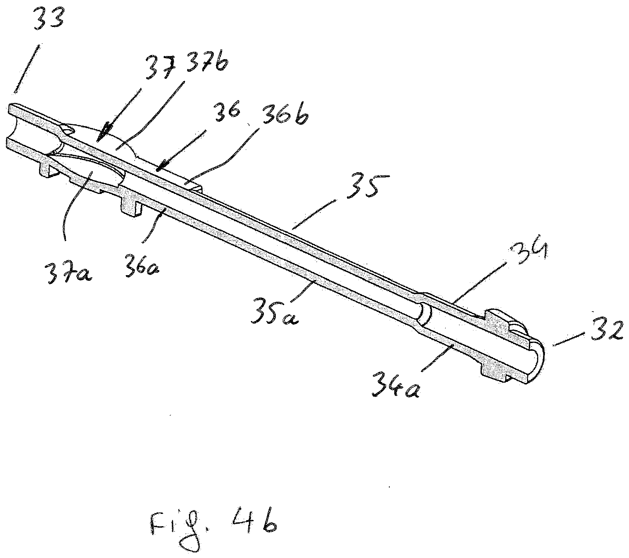

The infusion pump tube segment 30, which is individually shown in Fig. 4a and 4b in greater detail, is preferably made by the relatively new silicone injection molding production so that the accuracy tolerance is below 3% and its shape and form are kept during all infusion time since silicone does not change when squeezed by the follower heads 42a. Injection molded silicone tubes have also a better accuracy than standard extruded silicone tubes, that have large dimensional variations during extrusion.

Since in particular the injection silicone formed by a mold is used, the infusion pump tube segment 30 comprises several portions 34 to 37 that are adapted to a certain function as follows:
- Upstream, next to the inlet 32, there is a first tube portion 34 having a surrounding wall 34a with a reduced thickness (e.g. 0,5 mm) so that the upstream pressure sensor 20 can sense even the smallest suction in case of an upstream occlusion (reduction of the tube section) which is important to be sensed as early as possible.
- Subsequently, there is a normal second tube portion 35 having a thicker wall 35a (e.g. 1,0 mm) with an increased accuracy by higher suction so that it is reformed when the follower 42a is removed, so as to eliminate the need of a squeezing spring plate usually used in infusion pumps with an infusion pump tube segment made of PVC. This second tube portion 35 is not polished to reduce friction and power consumption.
- Subsequently, there is a third tube portion 36 having a polished square outer section 36c with flat walls 36b for a better and secure reading by air in line ultrasound sensors, as flat ultrasound plates engender best the flat sides of the tube.
- Subsequently, next to the outlet 33, there is a fourth tube portion 37 including a flat bottom compartment 37a having the shape of a disc with a larger diameter which compartment when pressurized exercises a higher force to the downstream pressure sensor 22 against it (F=P*S), a feature needed to sense accurately the downstream pressure from where important observations like the possibility of occlusion of the central venous catheter used by chronic patients are deduced.

An infusion pump infusion set anti-kicking tubing is connected through barb connectors (not shown) to the infusion pump tube segment 30 at each side, i.e. at its inlet 32 and at its outlet 33. Furthermore, said compartment 37a includes a small seed (not shown) inserted inside its cavity to reduce high pressure variations and oscillations and to increase accuracy wherein that part has a hard cover on the top for better pressure reading but also to secure a barb connector aside it.

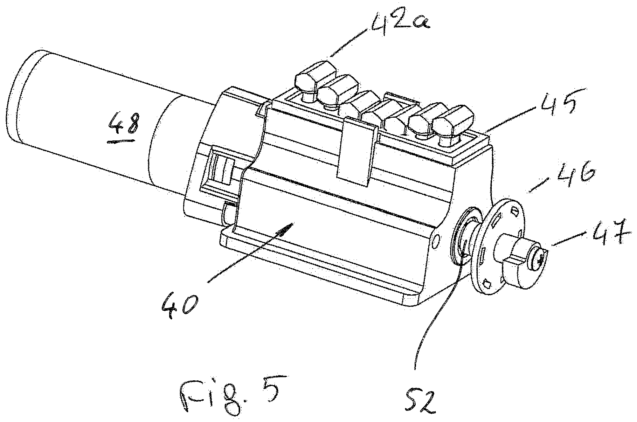

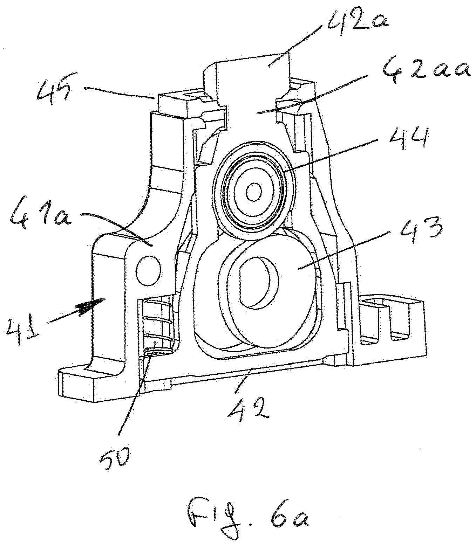

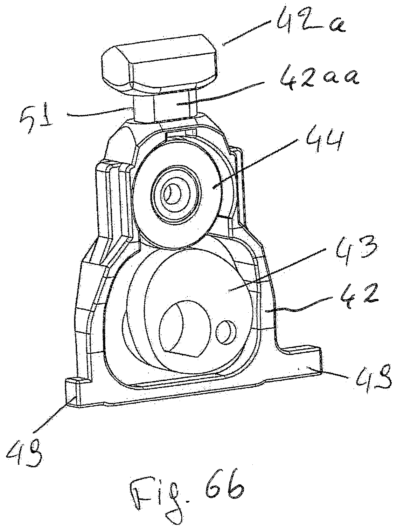

In the shown embodiment, the pump mechanism module 40, which is individually shown in Fig. 5 in greater detail, operates according to the linear peristaltic principle as basically known from EP 0 858 812 A1, and comprises a lot of follower units 41 arranged side by side. A preferred embodiment of a follower unit 41 is individually shown in Fig. 6a, and a follower which is part of the follower unit 41 is individually shown in Fig. 6b in greater detail wherein also the position of the rotating cam 43 inside it is also shown. The follower units 41 of the pump mechanism module 40 each operate with a reduced friction by means of a ball bearing 44 at a follower body 42 against an associated cam 43 which is arranged at a support 41a being part of the follower unit 41, wherein the follower body 42 is moveably mounted at said support 41a and comprises a follower head 42a provided on top of the follower body 42 (cf. Figs. 6a and b). The cam 43 rolls over the ball bearing 44 for low friction and, this way, the cam 43 can have a form to reduce noise and also causes a uniform load on a motor 48 shown in Fig. 5 which motor is a drive unit for the pump mechanism module 40. The cam 43 of each follower unit 41 is mounted on a common rotary shaft 52 (a part of which is shown in Fig. 5) which is rotated by the motor 48, wherein the rotational angle offset of the cam 43 relative to the rotary shaft 52 increases from follower unit 41 to follower unit 41 so that the follower heads 42a are temporally brought into engagement with the infusion pump tube segment 30 from follower unit 41 to follower unit 41.

The total design of the pump mechanism module 40 is for small size weight and friction while reducing noise to minimum. The follower bodies 42 comprise spring loading side walls so that they do not kick at the sides during infusion (noise reduction) and perform a removing follower action without friction using the same ball bearing 44 that causes the squeezing of the infusion tube segment 30 through the same follower head 42a. Each follower body 42 is provided with a runner 49 at each side where it slips without friction with the neighboring follower body 42 as to be seen from Fig. 6b. There is a spring 50 between the support 41a and follower body 42 as shown in Fig. 6a in order to remove the follower head 42a from squeezing the infusion pump tube segment 30 in addition to a ball bearing action.

In case a follower body 42 is blocked, the cam 43 at the removing follower cycle (after having squeezed the infusion pump tube segment 30) will do it but with friction, as there is no ball bearing at the back side. This is why a spring force which is subject to the infusion pump tube segment 30 causes a removement of a follower body 42 without friction for small blocks, whereas the use of the cam 43 is the last means to force the infusion pump tube segment 30 to open but at a friction price to pay.

The pump mechanism module 40 as shown in Fig. 5 forms one independent part that has only one rod for assembly and a screw on the other side to adjust the distance from a pressure plate, resulting in an operation under nominal pressure. In particular, the screw is adapted to adjust a squeezing gap in the infusion pump tube segment resulting in an adjustment of the maximum downstream occlusion pressure the infusion pump tube segment can withstand before leaking backwards, at nominal value. This way as contact points with the pump 1 body are minimal, a motor and mechanism noise is not transmitted through the body to the air, being a very important feature for infusion pumps.

The mechanism has a silicone liquid ingress sealing membrane 45 provided (not over the follower heads 42a as in prior art but) at an intermediate point 51 below the edge of the follower heads 42a, as to be seen from the figures 5 and 6a and b. The membrane 45 is preferably made by injection silicone molding and has orthogonal holes for a reduced section 42aa forming a stem and provided at each one of the follower bodies 42 at said point 51. The membrane 45 is very elastic and able to pass each follower head 42a from the hole water tightly, so that it is squeezed on the pump body by the pump mechanism module 40 for peripheral water tightness. A feedback for motor speed and infusion volume control is obtained by a first disc 46 for precise control (24 steps per turn) and a second disc 47 for rounds control also used as redundancy for safety control (cf. Fig. 5).

## Claims

1. A peristaltic infusion pump tube segment having an upstream inlet (32) and a downstream outlet (33) and adapted to be temporally squeezed at at least one squeezed point to be generated by an engagement structure and to be moved towards said outlet for passing a medical fluid from said inlet to said outlet,
comprising a first tube portion (34) next to said inlet (32) and having a surrounding wall (34a) which is adapted for an upstream occlusion detection,
a second tube portion (35) downstream of said first tube portion (34) and adapted to be temporally engaged by the engagement structure, wherein said second tube portion (35) comprises a surrounding wall (35a) made of an elastic material which allows said second tube portion (35) to be essentially reformed at those portions which are currently not subject to the engagement by the engagement structure,
a third tube portion (36) downstream of said second tube portion (35) and having a surrounding wall (36a) which is adapted for an air in line detection, and
a fourth tube portion (37) next to said outlet (33) and adapted for a downstream pressure detection,
**characterized in that**
the wall (34a) of said first tube portion (34) has a thickness which is dimensioned so as to allow a detection unit to detect even the smallest possible upstream occlusion, the wall (35a) of said second tube portion (35) is thicker than the wall (34a) of said first tube portion (34) and is essentially unpolished,the wall (36a) of said third tube portion (36) either comprises at least one flat wall portion (36b) which is configured for the air in line detection in particular by means of at least one ultrasound sensor, or has a rectangular or square section (36c) at least at its external side and comprises flat wall (36b) portions configured for the air in line detection in particular by means of at least one ultrasound sensor, wherein the outer surface of the at least one flat wall (36b) portion is polished, said fourth tube portion (37) comprises an essentially flat compartment (37a) configured for the downstream pressure detection, wherein said flat compartment (37a) comprises a disc-like element (37b) having essentially an at least part-circle or part-elliptical shape with a diameter or width being larger than the diameter or width of said first tube portion (34) and/or said second tube portion (35) and/or said third tube portion (36).

2. The peristaltic infusion pump tube segment according to claim 1, which is, at least partly, made of silicone, in particular by a silicone molding process, preferably by a silicone injection molding process.

3. The peristaltic infusion pump tube segment according to at least any one of the preceding claims, wherein the wall (35a) of said second tube portion (35) is configured to allow a viscous fluid to be passed through by suction effect.

4. The peristaltic infusion pump tube segment according to any one of the preceding claims, wherein said flat compartment (37a) comprises a long side extending in the direction of the peristaltic infusion pump tube segment (30).

5. An infusion pump device comprising a pump mechanism (40) configured as a peristaltic mechanism and including the peristaltic infusion pump tube segment (30) according to at least any one of the preceding claims, wherein the upstream inlet (32) of said peristaltic infusion pump tube segment (30) is adapted to be fluidly connected to an outlet (18) of a medication reservoir (14).

6. The infusion pump device according to claim 5, wherein said pump mechanism (40) is configured as a linear peristaltic mechanism with said peristaltic infusion pump tube segment (30) having an essentially elongated shape.

7. The infusion pump device according to claim 5 or 6, wherein said peristaltic infusion pump tube segment (30) is replaceable and/or disposable.

8. The infusion pump device according to at least any one of the claims 5 to 7, comprising an upstream pressure sensor (20) provided at said first tube portion (34).

9. The infusion pump device according to at least any one of the claims 5 to 8, wherein said pump mechanism (40) comprises an engagement structure adapted to generate at least one squeezed point in said second tube portion (35) and to move it in the direction towards said outlet (33) of said peristaltic infusion pump tube segment (30).

10. The infusion pump device according to the claims 6 and 9, wherein said engagement structure comprises a plurality of engagement units (41) arranged side by side along the length of said second tube portion (35) of said peristaltic infusion pump tube segment (30), wherein each engagement unit (41) comprises a follower head (42a) which is movable at an angle, in particular a right angle, relative to said second tube portion (35) and adapted to be brought into engagement with said second tube portion (35) in order to squeeze it, wherein said engagement structure is further configured so that the follower heads (42a) are temporally brought into engagement with said second tube portion (35) one after another.

11. The infusion pump device according to claim 10, wherein at least one engagement unit (41) comprises a support (41a), a follower body (42) moveably mounted at said support (41a) and provided with the follower head (42a), a ball bearing (44) rotatably mounted at said follower body (42), a cam (43) rotatably mounted at said support (41a) and in sliding engagement with said ball bearing (44), and a spring (50) biasing said moveable follower body (42) with said follower head (42a) away from said second tube portion (35) of said peristaltic infusion pump tube segment (30).

12. The infusion pump device according to claim 11, wherein said engagement structure comprises a drive unit (48), wherein the cam (43) of each equipment unit (41) is fixedly mounted to a common rotary shaft (52) which is rotated by said drive unit (48), and wherein the rotational angle offset of the cam (43) relative to said rotary shaft (52) increases from engagement unit (41) to engagement unit (41) so that the follower heads (42a) are temporally brought into engagement with said second tube portion (35) of said peristaltic infusion pump tube segment (30) from engagement unit (41) to engagement unit (41).

13. The infusion pump device according to claim 11 or 12, wherein the follower head (42a) is mounted upon a stem (42aa) fixed to the follower body (42) and the engagement unit (41) further comprises a sealing membrane (45) with an opening through which said stem (42aa) extends in an at least essentially sealing manner.

14. The infusion pump device according to at least any one of the claims 5 to 13, comprising a first air in line sensor (22), in particular an air in line ultrasound sensor, provided at said third tube portion (36).

15. The infusion pump device according to at least any one of the claims 5 to 14, comprising a downstream pressure sensor (24) provided at said fourth tube portion (37).

16. The infusion pump device according to at least any one of the claims 5 to 15, comprising an air eliminating filter which is in fluid communication with the outlet (33) of said peristaltic infusion pump tube segment (30) and a second air in line sensor (26) adapted to detect air in a fluid path downstream of said air eliminating filter.

## Patentansprüche

1. Peristaltisches Infusionspumpenschlauchsegment mit einem stromaufwärtigen Einlass (32) und einem stromabwärtigen Auslass (33), welches so ausgelegt ist, an mindestens einem durch eine Eingriffsstruktur zu erzeugenden und in Richtung des Auslasses zu bewegenden Quetschpunkt vorübergehend zusammengedrückt zu werden, um eine medizinische Flüssigkeit vom Einlass zum Auslass zu leiten,
mit einem am nächsten zum Einlass (32) gelegenen ersten Schlauchabschnitt (34) mit einer umgebenden Wand (34a), die für eine stromaufwärtige Okklusionserkennung ausgelegt ist,
einem zweiten Schlauchabschnitt (35) stromabwärts des ersten Schlauchabschnitts (34), der dazu ausgelegt ist, vorübergehend von der Eingriffsstruktur in Eingriff genommen zu werden, wobei der zweite Schlauchabschnitt (35) eine umgebende Wand (35a) aus einem elastischen Material aufweist, das es ermöglicht, den zweiten Schlauchabschnitt (35) an denjenigen Abschnitten, die derzeit nicht dem Eingriff durch die Eingriffsstruktur unterliegen, im Wesentlichen wieder in seine ursprüngliche Form zu bringen,
einem dritten Rohrabschnitt (36) stromabwärts des zweiten Rohrabschnitts (35) mit einer umgebenden Wand (36a), die für eine Luft-in-der-Leitung-Erkennung ausgelegt ist, und
einem am nächsten zum Auslass (33) gelegenen vierten Rohrabschnitt (37), der für eine stromabwärtige Druckerkennung ausgelegt ist,
**dadurch gekennzeichnet, dass**
die Wand (34a) des ersten Rohrabschnitts (34) eine Dicke aufweist, die so dimensioniert ist, dass eine Detektionseinheit selbst die geringstmögliche stromaufwärtige Verstopfung erkennen kann, die Wand (35a) des zweiten Rohrabschnitts (35) dicker ist als die Wand (34a) des ersten Rohrabschnitts (34) und im Wesentlichen unpoliert ist, die Wand (36a) des dritten Rohrabschnitts (36) entweder mindestens einen flachen Wandabschnitt (36b) aufweist, der für die Luft-in-der-Leitung-Erkennung insbesondere mittels mindestens eines Ultraschallsensors konfiguriert ist oder zumindest an ihrer Außenseite einen rechteckigen oder quadratischen Querschnitt (36c) besitzt und flache Wandabschnitte (36b) aufweist, die für die Luft-in-der-Leitung-Erkennung insbesondere mittels mindestens eines Ultraschallsensors konfiguriert sind, wobei die Außenfläche des mindestens einen flachen Wandabschnitts (36b) poliert ist, der vierte Rohrabschnitt (37) ein im Wesentlichen flaches Kompartment (37a) aufweistt, das für die Erfassung des stromabwärtigen Drucks konfiguriert ist, wobei das flache Kompartment (37a) ein scheibenartiges Element (37b) aufweist, das im Wesentlichen eine zumindest teilkreisförmige oder teilelliptische Form mit einem Durchmesser oder einer Breite hat, der bzw. die größer ist als der Durchmesser oder die Breite des ersten Rohrabschnitts (34) und/oder des zweiten Rohrabschnitts (35) und/oder des dritten Rohrabschnitts (36).

2. Peristaltisches Infusionspumpenschlauchsegment nach Anspruch 1, das zumindest teilweise aus Silikon hergestellt ist, insbesondere durch ein Silikonformverfahren, vorzugsweise durch ein Silikonspritzgussverfahren.

3. Peristaltisches Infusionspumpenschlauchsegment nach mindestens einem der vorstehenden Ansprüche, wobei die Wand (35a) des zweiten Schlauchabschnitts (35) so konfiguriert ist, dass eine viskose Flüssigkeit durch einen Saugeffekt hindurchgeleitet werden kann.

4. Peristaltisches Infusionspumpenschlauchsegment nach einem der vorstehenden Ansprüche, wobei das flache Kompartment (37a) eine Längsseite aufweist, die sich in Richtung des peristaltischen Infusionspumpenschlauchsegments (30) erstreckt.

5. Infusionspumpvorrichtung, die einen Pumpmechanismus (40) umfasst, der als peristaltischer Mechanismus ausgebildet ist und das peristaltische Infusionspumpenschlauchsegment (30) nach mindestens einem der vorstehenden Ansprüche enthält, wobei der stromaufwärtige Einlass (32) des peristaltischen Infusionspumpenschlauchsegments (30) so ausgelegt ist, dass er fluidisch mit einem Auslass (18) eines Medikamentenreservoirs (14) verbunden werden kann.

6. Infusionspumpvorrichtung nach Anspruch 5, wobei der Pumpmechanismus (40) als linearer peristaltischer Mechanismus ausgebildet ist, wobei das peristaltische Infusionspumpenschlauchsegment (30) eine im Wesentlichen längliche Form aufweist.

7. Infusionspumpvorrichtung nach Anspruch 5 oder 6, wobei das peristaltische Infusionspumpenschlauchsegment (30) austauschbar und/oder wegwerfbar ist.

8. Infusionspumpvorrichtung nach mindestens einem der Ansprüche 5 bis 7, welche einen stromaufwärts angeordneten Drucksensor (20) aufweist, der an dem ersten Schlauchabschnitt (34) vorgesehen ist.

9. Infusionspumpvorrichtung nach mindestens einem der Ansprüche 5 bis 8, wobei der Pumpmechanismus (40) eine Eingriffsstruktur aufweist, die dazu ausgelegt ist, mindestens einen Quetschpunkt in dem zweiten Schlauchabschnitt (35) zu erzeugen und diesen in Richtung des Auslasses (33) des peristaltischen Infusionspumpenschlauchsegments (30) zu bewegen.

10. Infusionspumpvorrichtung nach den Ansprüchen 6 und 9, wobei die Eingriffsstruktur eine Vielzahl von Eingriffseinheiten (41) aufweist, die nebeneinander entlang der Länge des zweiten Schlauchabschnitts (35) des peristaltischen Infusionspumpenschlauchsegments (30) angeordnet sind, wobei jede Eingriffseinheit (41) einen Mitnehmerkopf (42a) aufweist, der in einem Winkel, insbesondere einem rechten Winkel, relativ zum zweiten Schlauchabschnitt (35) bewegbar ist und mit dem zweiten Schlauchabschnitt (35) in Eingriff gebracht werden kann, um diesen zusammenzudrücken, wobei die Eingriffsstruktur ferner so konfiguriert ist, dass die Mitnehmerköpfe (42a) zeitlich nacheinander mit dem zweiten Schlauchabschnitt (35) in Eingriff gebracht werden.

11. Infusionspumpvorrichtung nach Anspruch 10, wobei mindestens eine Eingriffseinheit (41) eine Halterung (41a), einen an der Halterung (41a) beweglich gelagerten Mitnehmerkörper (42) mit einem Mitnehmerkopf (42a), ein an dem Mitnehmerkörper (42) drehbar gelagertes Kugellager (44), eine Nocke (43), die drehbar an der Halterung (41a) angebracht ist und in Gleitverbindung mit dem Kugellager (44) steht, und eine Feder (50) umfasst, die den beweglichen Mitnehmerkörper (42) mit dem Mitnehmerkopf (42a) von dem zweiten Rohrabschnitt (35) des peristaltischen Infusionspumpenrohrsegments (30) weg vorspannt.

12. Infusionspumpvorrichtung nach Anspruch 11, wobei die Eingriffsstruktur eine Antriebseinheit (48) aufweist, wobei der Nocken (43) jeder Ausrüstungseinheit (41) fest an einer gemeinsamen Drehwelle (52) angebracht ist, die von der Antriebseinheit (48) gedreht wird, und wobei der Drehwinkelversatz des Nockens (43) relativ zu der Drehwelle (52) von Ausrüstungseinheit (41) zu Ausrüstungseinheit (41) zunimmt, so dass die Mitnehmerköpfe (42a) vorübergehend in Eingriff mit dem zweiten Schlauchabschnitt (35) des peristaltischen Infusionspumpenschlauchsegments (30) von Ausrüstungseinheit (41) zu Ausrüstungseinheit (41) gebracht werden.

13. Infusionspumpvorrichtung nach Anspruch 11 oder 12, wobei der Mitnehmerkopf (42a) auf einem Stab (42aa) montiert ist, der am Mitnehmerkörper (42) befestigt ist, und die Eingriffseinheit (41)ferner eine Dichtungsmembran (45) mit einer Öffnung aufweist, durch die sich der Stab (42aa) in einer zumindest im Wesentlichen abdichtenden Weise erstreckt.

14. Infusionspumpvorrichtung nach mindestens einem der Ansprüche 5 bis 13, welche einen ersten Luft-in-der-Leitung-Sensor (22), insbesondere einen Luft-in-der-Leitung-Ultraschallsensor, aufweist, der an dem dritten Schlauchabschnitt (36) vorgesehen ist.

15. Infusionspumpvorrichtung nach mindestens einem der Ansprüche 5 bis 14, welche einen stromabwärts gelegenen Drucksensor (24) aufweist, der an dem vierten Schlauchabschnitt (37) vorgesehen ist.

16. Infusionspumpvorrichtung nach mindestens einem der Ansprüche 5 bis 15, welche einen Luftentfernungsfilter, der in Fluidverbindung mit dem Auslass (33) des peristaltischen Infusionspumpenschlauchabschnitts (30) steht, und einen zweiten Luft-in-der-Leitung-Sensor (26), der dazu ausgelegt ist, Luft in einem Fluidweg stromabwärts des Luftentfernungsfilters zu erfassen, aufweist.

## Revendications

1. Segment de tube de pompe à perfusion péristaltique présentant une entrée amont (32) et une sortie aval (33) et conçu pour être temporairement comprimé au niveau d'au moins un point de pincement devant être généré par une structure de mise en prise et pour être déplacé en direction de ladite sortie afin de faire passer un fluide médical de ladite entrée à ladite sortie,
comprenant une première partie de tube (34) à côté de ladite entrée (32) et ayant une paroi environnante (34a) qui est adaptée à la détection d'occlusion en amont, une deuxième partie de tube (35) en aval de ladite première partie de tube (34) et conçue pour être temporairement mise en prise par la structure de mise en prise, dans lequel ladite deuxième partie de tube (35) comprend une paroi environnante (35a) composée d'un matériau élastique qui permet à ladite deuxième partie de tube (35) d'être sensiblement reformée au niveau des endroits qui ne sont actuellement pas soumis à la mise en prise par la structure de mise en prise,
une troisième partie de tube (36) en aval de ladite deuxième partie de tube (35) et présentant une paroi environnante (36a) qui est conçue pour une détection d'air dans une conduite, et
une quatrième partie de tube (37) à côté de ladite sortie (33) et conçue pour une détection de pression en aval,
**caractérisé en ce que**
la paroi (34a) de ladite première partie de tube (34) présente une épaisseur qui est dimensionnée de manière à permettre à une unité de détection de détecter même la plus petite occlusion amont possible, la paroi (35a) de ladite deuxième partie de tube (35) étant plus épaisse que la paroi (34a) de ladite première partie de tube (34) et étant essentiellement non polie, la paroi (36a) de ladite troisième partie de tube (36) comprend soit au moins une partie de paroi plate (36b) qui est configurée pour la détection d'air dans une conduite notamment au moyen d'au moins un capteur à ultrasons, ou présente une section rectangulaire ou carrée (36c) au moins sur son côté externe et comprend des parties de paroi plate (36b) configurées pour la détection d'air dans une conduite notamment au moyen d'au moins un capteur à ultrasons, dans lequel la surface externe de l'au moins une partie de paroi plate (36b) est polie, ladite quatrième partie de tube (37) comprend un compartiment sensiblement plat (37a) configuré pour la détection de pression en aval, dans lequel ledit compartiment plat (37a) comprend un élément de type disque (37b) présentant sensiblement une forme au moins partiellement circulaire ou partiellement elliptique avec un diamètre ou une largeur supérieur(e) au diamètre ou à la largeur de ladite première partie de tube (34) et/ou de ladite deuxième partie de tube (35) et/ou de ladite troisième partie de tube (36).

2. Segment de tube de pompe à perfusion péristaltique selon la revendication 1, qui est, au moins en partie, composé de silicone, en particulier par un procédé de moulage de silicone, de préférence par un procédé de moulage par injection de silicone.

3. Segment de tube de pompe à perfusion péristaltique selon au moins l'une quelconque des revendications précédentes, dans lequel la paroi (35a) de ladite deuxième partie de tube (35) est configurée pour permettre le passage d'un fluide visqueux par effet d'aspiration.

4. Segment de tube de pompe à perfusion péristaltique selon l'une quelconque des revendications précédentes, dans lequel ledit compartiment plat (37a) comprend un côté long s'étendant dans la direction du segment de tube de pompe à perfusion péristaltique (30).

5. Dispositif de pompe à perfusion comprenant un mécanisme de pompe (40) configuré sous la forme d'un mécanisme péristaltique et comportant le segment de tube de pompe à perfusion péristaltique (30) selon au moins l'une quelconque des revendications précédentes, dans lequel l'entrée amont (32) dudit segment de tube de pompe à perfusion péristaltique (30) est conçue pour être reliée fluidiquement à une sortie (18) d'un réservoir de médicament (14).

6. Dispositif de pompe à perfusion selon la revendication 5, dans lequel ledit mécanisme de pompe (40) est configuré sous la forme d'un mécanisme péristaltique linéaire avec ledit segment de tube de pompe à perfusion péristaltique (30) présentant une forme sensiblement allongée.

7. Dispositif de pompe à perfusion selon la revendication 5 ou 6, dans lequel ledit segment de tube de pompe à perfusion péristaltique (30) est remplaçable et/ou jetable.

8. Dispositif de pompe à perfusion selon au moins l'une quelconque des revendications 5 à 7, comprenant un capteur de pression amont (20) disposé au niveau de ladite première partie de tube (34).

9. Dispositif de pompe à perfusion selon au moins l'une quelconque des revendications 5 à 8, dans lequel ledit mécanisme de pompe (40) comprend une structure de mise en prise conçue pour générer au moins un point de pincement dans ladite deuxième partie de tube (35) et pour déplacer celui-ci en direction de ladite sortie (33) dudit segment de tube de pompe à perfusion péristaltique (30).

10. Dispositif de pompe à perfusion selon les revendications 6 et 9, dans lequel ladite structure de mise en prise comprend une pluralité d'unités de mise en prise (41) agencées côte à côte le long de la longueur de ladite deuxième partie de tube (35) dudit segment de tube de pompe à perfusion péristaltique (30), dans lequel chaque unité de mise en prise (41) comprend une tête suiveuse (42a) qui est mobile selon un angle, en particulier un angle droit, par rapport à la deuxième partie de tube (35) et conçue pour être mise en prise avec ladite deuxième partie de tube (35) afin de la comprimer, dans lequel ladite structure de mise en prise est en outre configurée de sorte que les têtes suiveuses (42a) soient mises temporairement en prise avec ladite deuxième partie de tube (35) l'une après l'autre.

11. Dispositif de pompe à perfusion selon la revendication 10, dans lequel au moins une unité de mise en prise (41) comprend un support (41a), un corps suiveur (42) monté de façon mobile au niveau dudit support (41a) et doté de la tête suiveuse (42a), un roulement à billes (44) monté en rotation au niveau dudit corps suiveur (42), une came (43) montée en rotation au niveau dudit support (41a) et en prise coulissante avec ledit roulement à billes (44), et un ressort (50) sollicitant ledit corps suiveur mobile (42) avec ladite tête suiveuse (42a) à l'écart de ladite deuxième partie de tube (35) dudit segment de tube de pompe à perfusion péristaltique (30).

12. Dispositif de pompe à perfusion selon la revendication 11, dans lequel ladite structure de mise en prise comprend une unité d'entraînement (48), dans lequel la came (43) de chaque unité d'équipement (41) est fixement montée sur un arbre rotatif commun (52) qui est entraîné en rotation par ladite unité d'entraînement (48), et dans lequel le décalage d'angle de rotation de la came (43) par rapport audit arbre rotatif (52) augmente d'unité de mise en prise (41) à unité de mise en prise (41) de sorte que les têtes suiveuses (42a) soient temporairement mises en prise avec ladite deuxième partie de tube (35) dudit segment de tube de pompe à perfusion péristaltique (30) d'unité de mise en prise (41) à unité de mise en prise (41).

13. Dispositif de pompe à perfusion selon la revendication 11 ou 12, dans lequel la tête suiveuse (42a) est montée sur une tige (42aa) fixée au corps suiveur (42) et l'unité de mise en prise (41) comprend en outre une membrane d'étanchéité (45) avec une ouverture à travers laquelle ladite tige (42aa) s'étend d'une manière au moins sensiblement étanche.

14. Dispositif de pompe à perfusion selon au moins l'une quelconque des revendications 5 à 13, comprenant un premier capteur (22) d'air dans une conduite, en particulier un capteur ultrasonique d'air dans une conduite, disposé au niveau de ladite troisième partie de tube (36).

15. Dispositif de pompe à perfusion selon au moins l'une quelconque des revendications 5 à 14, comprenant un capteur de pression aval (24) disposé au niveau de ladite quatrième partie de tube (37).

16. Dispositif de pompe à perfusion selon au moins l'une quelconque des revendications 5 à 15, comprenant un filtre d'élimination d'air qui est en communication fluidique avec la sortie (33) dudit segment de tube de pompe à perfusion péristaltique (30) et un second capteur (26) d'air dans une conduite conçu pour détecter de l'air dans un trajet de fluide en aval dudit filtre d'élimination d'air.
